# EUROPEAN PATENT SPECIFICATION

(11) **EP 4 101 959 B1**
(45) Date of publication and mention of the grant of the patent: **07.05.2025**
(21) Application number: 20923869.0
(22) Date of filing: 18.06.2020
(51) Int. Cl.: A61F 2/06, D03D 13/00, D03D 1/00, D06B 23/02, D06C 7/02, D06C 23/04

(54) **ARTIFICIAL BLOOD VESSEL AND PREPARATION METHOD THEREFOR**
KÜNSTLICHES BLUTGEFÄSS UND VERFAHREN ZU SEINER HERSTELLUNG
VAISSEAU SANGUIN ARTIFICIEL ET PROCÉDÉ DE PRÉPARATION ASSOCIÉ

(30) Priority: 11.03.2020 CN 202010167879
(43) Date of publication of application: 14.12.2022
(73) Proprietor: ZHEJIANG ACCUPATH SMART MANUFACTURING (GROUP) CO., LTD., Jiaxing, Zhejiang 314006 (CN)
(72) Inventor: MING, Jing, Jiaxing, Zhejiang 314006 (CN); MA, Xiaoman, Jiaxing, Zhejiang 314006 (CN); GU, Ziqi, Jiaxing, Zhejiang 314006 (CN); ZHANG, Hui, Jiaxing, Zhejiang 314006 (CN); ZHANG, Jing, Jiaxing, Zhejiang 314006 (CN)
(74) Representative: Patentship Patentanwaltsgesellschaft mbH
(86) International application number: PCT/CN2020/096804
(87) International publication number: WO 2021/179468

(56) References cited:
- CN-A- 101 084 846
- CN-A- 105 030 372
- CN-A- 105 105 867
- CN-A- 105 105 867
- CN-A- 105 963 051
- CN-A- 110 604 633
- CN-U- 86 207 175
- JP-A- H10 277 068
- US-A- 4 530 113
- US-A- 5 127 919

## Description

### TECHNICAL FIELD

The present invention relates to the field of medical devices and, in particular, to an artificial blood vessel and a method of preparing the same.

### BACKGROUND

With the aging of the population, the incidence rate of vascular diseases such as aortic aneurysm and aortic dissection have been greatly rising. Vascular reconstruction accomplished by resection and endovascular exclusion can greatly prolong patients' life. An aortic dissection (AD) is a condition in which blood flowing inside the aortic lumen enters the aortic media via a teared aortic intima and further tears the media tissue longitudinally along the aorta, thus forming a false lumen in addition to the real lumen. ADs are divided into De-Bake types I, II and III according to where the intimal tears originate. They all feature a dangerous onset and a high mortality rate. An artificial blood vessel sutured with a metal stent thereon, i.e., an intraoperative stent, may be delivered to the lesion site through open surgery, and the artificial blood vessel is secured without suture at both ends of the lesion artery by elastic expansion force of the stent, thus completely sealing the intimal breach. At the same time, a portion of the artificial blood vessel not sutured to the stent (about 1 cm long) is sutured to the aortic arch, thus achieving the purpose of AD treatment.

Research on woven artificial blood vessels has started since Voorhees observed the phenomenon of cells growing on a silk thread immersed in blood in 1952. As far, traditional textile techniques such as weaving, knitting and non-weaving remain mainstream methods for preparing artificial blood vessels. Materials of artificial blood vessels have experienced several generations of changes from metals and Vinyon-N to today's polyesters, silk, polytetrafluoroethylene, etc. The selection of appropriate fiber materials and textile structures has always been a research focus of domestic and foreign experts and scholars. Woven artificial blood vessels involve basic tissues of textile weaves such as plain weave, twill weave and satin weave or various variants thereof. Among them, plain weave artificial blood vessels are widely used because of their compact tube wall structure and small deformation, together with their minimum water (blood) permeability. However, low softness of such blood vessels will make it difficult to operate and suture during surgery, and even cause cell adhesion and growth difficulty, which is not conductive to the endothelialization of cells. Finally, it will lead to vascular blockage and other problems.

US 5 127 919 A discloses a vascular graft prosthesis of woven synthetic yarn where selected fill threads are woven into S-shaped lock elements about selected warp threads to provide a tubular fabric that resists fraying when cut at an oblique angle. US 4 530 113 A discloses graft prostheses woven with cross-weave patterns to prevent unravelling and to increase suture hold strength. CN 105 105 867 A discloses an integrally-molded woven blood vessel covered stent and a preparation method thereof.

### SUMMARY

The invention is set out in the appended set of claims.

It is an object of the present invention to provide an artificial blood vessel and a method of preparing the same, which can overcome the problems of low softness and excessive permeability of conventional artificial blood vessels.

To this end, the present invention provides a method of preparing an artificial blood vessel as defined in the appended claim 1.

Optionally, in the method, an area ratio of the first weave to the second weave may be from 1: 1 to 1: 3.

Optionally, the method may further include sizing the warp yarns, before the warp and weft yarns are woven into the composite fabric.

Before the composite fabric is corrugated, the method may further include desizing the composite fabric.

Optionally, in the method, the warp yarns may be sized at a sizing rate of 50 to 100 m/min and a drying cylinder temperature of 50 °C to 80 °C.

Optionally, in the method, the desizing may be accomplished at a temperature within the range of 80 °C to 85 °C for 3 h to 5 h.

Optionally, in the method, the warp and weft yarns may be spun yarns made of polymer materials with biocompatibility.

Optionally, in the method, a material of the spun yarns may be at least one selected from the group of polyethylene terephthalate, polyethylene, polytetrafluoroethylene and natural mulberry silk.

Optionally, in the method, the weft yarns may be selected from the group of monofilament, multiple wound yarns and twisted yarns.

The present invention also provides an artificial blood vessel as defined in appended claim 8.

Optionally, in the artificial blood vessel, an area ratio of the first weave to the second weave may be from 1: 1 to 1: 3**.**

In summary, the present invention provides an artificial blood vessel and a method of preparing the same. In the composite structure of the artificial blood vessel with the alternating first and second weaves formed by the warp and weft yarns according to the independent claims, a softness degree of the first weave is lower than that of the second weave. As a result, combining stiffness of the first weave with softness of the second weave, this composite structure ensures both low permeability and increased softness of the fabric. Therefore, it is improved to a certain extent over the conventional woven artificial blood vessels and is easier to handle and suture.

### BRIEF DESCRIPTION OF THE DRAWINGS

Fig. 1 is a flow diagram of a method for preparing an artificial blood vessel according to an embodiment of the present invention;
Fig. 2 schematically illustrates a composition of a composite fabric prepared in Embodiment 1 of the present invention;
Fig. 3 schematically illustrates a composition of a composite fabric prepared in Embodiment 2 of the present invention; and
Fig. 4 schematically illustrates a composition of a composite fabric prepared in Embodiment 3 of the present invention.

### DETAILED DESCRIPTION

The present invention is mainly to solve the problems of low softness and excessive permeability of conventional artificial blood vessels.

In view of the above, the present invention provides an artificial blood vessel and a method for preparing the same. The method includes: weaving warp and weft yarns to form a composite fabric with alternating plain and satin weaves; and successively corrugating and thermal-setting the composite fabric to obtain an artificial blood vessel. The artificial blood vessel adopts a composite structure of the plain and satin weaves formed by the warp and weft yarns, which not only ensures low permeability but improves the softness of the fabric. Therefore, the artificial blood vessel obtained in the present application has certain advantages over conventional woven artificial blood vessels and is easier to operate and suture.

The artificial blood vessel and method proposed by the present invention will be described in detail below with reference to the accompanying figures and specific embodiments. Note that the figures are provided in a very simplified form not necessarily drawn to exact scale and for the only purpose of facilitating easy and clear description of the embodiments. In addition, the structures shown in the figures are usually partially representations of their actual counterparts. In particular, as the figures would have different emphases, they are sometimes drawn to different scales.

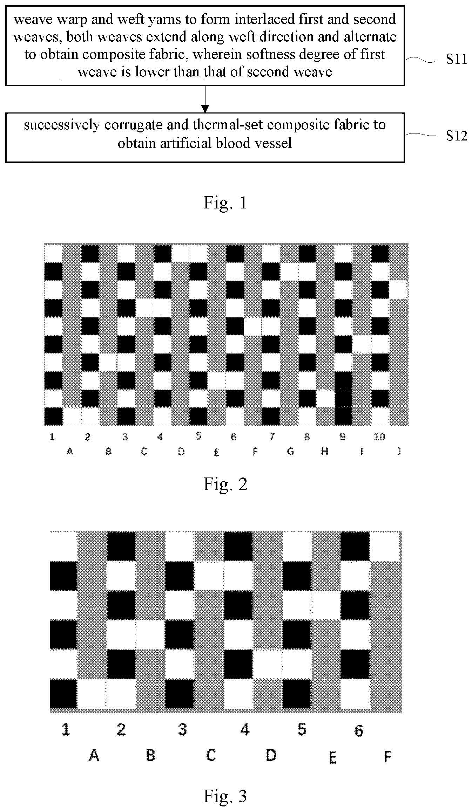

As shown in Fig. 1, according to an embodiment of the present invention, the method for preparing an artificial blood vessel includes the following steps:
S11) weaving warp and weft yarns to form interlaced first and second weaves, both weaves extend along a weft direction and alternate to obtain a composite fabric, wherein a softness degree of the first weave is lower than that of the second weave; and
S12) successively corrugating and thermal-setting the composite fabric to obtain the artificial blood vessel.

To make the composite fabric to meet the requirements of low permeability and high softness, in step S11, an area ratio of the first weave to the second weave may be 1: 1 to 1: 3. In particular, the area ratio may be 1: 1, 1: 2, 1: 3 or the like.

In this embodiment, the first weave is a plain weave and the second weave is a satin weave. The characteristic of plain weave is that warp yarns and weft yarns are interwoven with each other, and the interweaving of two adjacent yarns is just the opposite. Interweaving points of each yarn are exactly on the opposite sides of respective interweaving points of each adjacent yarn. Compared with other weaves, the plain weave has the most interweaving points. Hence, the plain weave endows the fabric a stiff and smooth surface and hand feeling. The characteristic of satin weave is that the warp and weft yarns will form some independent and unconnected weave points. Compared with other weaves, the stain weave has less interweaving points. Most of the warp (weft) yarns float on the weft (warp) yarns, and long floating warp (weft) lines are visible on the fabric surface. Hence, the satin weave endows the fabric soft hand feeling but prone to being snagged and pilled. In view of the above, combining a plain weave with a satin weave can result in a fabric with uniform long yarn floating lines on the surface, which endows the fabric a relatively soft feeling and is not prone to being snagged and pilled.

In some examples, the warp and weft yarns are implemented as spun yarns of a biocompatible polymer material. For example, a material of the warp and weft yarns is selected from the following group: polyethylene terephthalate, polyethylene, polytetrafluoroethylene and natural mulberry silk.

A fineness of the warp yarns may be 4.44 tex (40 D) to 11.11 tex (100 D). The warp yarns may be multifilament yarns each composed of different numbers of monofilament yarns. In one example, before the warp and weft yarns are woven into the composite fabric, the warp yarns are sized to form a uniform size layer coated on the surface, the sizing is conducted at a sizing rate of 50-100 m/min and a drying cylinder temperature of 50 °C to 80 °C. The size layer would increase the strength and friction resistance of warp yarns. After that, the sized warp yarns may be further subjected to sectional warping.

Since there is no need for the weft yarns to pass through heddle eyes or reed dents during the weaving process, they are less worn and do not need to be sized. Therefore, the weft yarns may be selected from the group of monofilament, multiple wound yarns and twisted yarns. The fineness of the weft yarns may range from 4.44 tex (40 D) to 11.11 tex (100 D). The number of single yarns in multiple yarns may range from 0 to 6, and a twist level thereof may range from 0 to 300 turns per 10 cm. After being twisted, torsion between fibers within the yarns will increase, and distance between fibers will also increase with the increase of its twist level. As a result, when aforementioned warp yarns and weft yarns interlaced with each other, a porosity of the resulting fabric will increase accordingly. Therefore, permeability of a fabric has a close relationship with the twist level of the used yarns. Hence, it is suggested to select yarns with appropriate twist level according to practical needs. In one example, before the warp and weft yarns are woven to form the composite fabric, the weft yarns are subject to a winding or twisting operation on a winder or twister. They are then evenly wound on a shuttle and thus get ready to be woven. If the weft yarns are selected to be monofilament yarns, the number of single yarns in wound yarns would be set to zero for the winder. In another example, there is no need for the weft yarns to be twisted, then the twist level would be set to zero for the twister.

The warp and weft yarns may be woven on a high-precision weaving machine. After the warp yarns are unrolled from a warp beam, the warp yarns further undergo a sequential threading treatment, i.e., the warp yarns sequentially pass through heddle eyes and reed dents. After that, the warp yarns interlace with the weft yarns to form the composite fabric with interlaced plain and satin weaves.

During the weaving process, the warp yarns will receive repeated friction from heddle eyes, reed dents and shuttle. The friction makes the warp yarns prone to being snagged and pilled, and causes warp yarns to break, resulting in obvious defects on the fabric surface. Consequently, after subsequent corrugating and setting treatment, the appearance of artificial blood vessels presents defects such as lousiness, resulting in an uneven tube wall thereof, which may have an adverse effect on treatment effect when using the artificial blood vessel. In order to avoid this, according to embodiments of the present application, before the warp and weft yarns are woven to form the composite fabric, i.e., prior to the warp yarns treading through the heddle eyes, reed dents and shuttle successively, the warp yarns are sized to ensure the process goes smooth and finally enable the resulting artificial blood vessel to have an even tube wall.

After the warp yarns are sized in step S11 and before the composite fabric is corrugated in step S12, the composite fabric is desized. In one example, the de-sizing is accomplished at a temperature in the range of 80 °C to 85 °C for a period of time ranging from 3 h to 5 h.

During the corrugation of the composite fabric in step 12, the desized composite fabric may be disposed on a stainless steel core shaft with a pitch of 1 to 2 mm, and a thick-denier high-strength yarn may be then wound thereon with a tension of 10 kg/N to 12 kg/N. During the setting process, the core shaft with the wound fabric thereon may be disposed in hot water at a temperature of 80-90 °C for 3-5 min to stabilize the corrugated shape of the fabric, and then dried. In this way, the artificial blood vessel with high softness and low permeability can be obtained.

Embodiments of the present application also provide an artificial blood vessel, which is prepared by the methods above. The artificial blood vessel is a three-dimensional tube obtained from a thermal setting process and having a diameter of 8 mm to 36 mm. Further, according to the invention, the composite fabric of the artificial blood vessel has a warp density ranging from 480 yarns per 10 cm to 748 yarns per 10 cm and a weft density ranging from 280 yarns per 10 cm to 400 yarns per 10 cm, which ensure that the artificial blood vessel has desirable softness and low permeability.

In order to enable the composite fabric to have both low permeability and high softness, in some examples, the area ratio of the first weave to the second weave is 1: 1, the first weave is a plain weave, and the second weave is a satin weave with warps floating over 5 to 9 wefts. The artificial blood vessel and method according to the present application will be explained in detail below by way of the exemplary examples. Advantages and objects of the present application will be more apparent from the description of the following examples.

### Embodiment 1

The warp yarns were 8.89 tex/24F (80D/24F) medical grade polyethylene terephthalate yarns, which were multifilament yarns each composed of 24 monofilament yarns and had been sized. The weft yarns were twisted yarns wherein 3 monofilaments were twisted at a level of 300 turns per 10 cm, each made of 4.44 tex (40D) polyester. A composite fabric was made of plain weaves and satin weaves with 9 long floating lines, wherein the area ratio of the plain weaves to the satin weaves was 1: 1. The composite fabric was further processed into a fabric tube with a diameter of 24 mm.

The appearance of the composite fabric is shown in Fig. 2, wherein black blocks represent warp interlacing points of the plain weave, gray blocks represent warp interlacing points of the satin weave. Moreover, the number of long warp floating lines was 9, and white blocks represent weft interlacing points. The Arabic numerals 1, 2, 3 ... 10 denote the plain weave, and the capital letters A, B, C ... J denote the satin weave.

The fabric for the artificial blood vessel was woven on a high-precision weaving machine, and had a warp density of 600 yarns per 10 cm and a weft density of 280 yarns per 10 cm. The total number of the warp yarns was 450, each has 24 fibers, and warp beams of two systems operated simultaneously to feed the warp yarns. A sequential threading process followed, which involved threading the warp yarns through a total of 60 reed dents successively. During the weaving process, the yarns were weaved with even tension, and no breakage or pilling occurred. In this way, the resulting composite fabric had noticeable floating lines, a uniform and neat texture, and glossy and clean appearance.

The composite fabric was subject to desizing, corrugating and thermal setting processes in sequence. The desizing process was carried out at a temperature of 85 °C for 5 h. After the desizing process, the composite fabric was disposed on a stainless steel core shaft with a pitch of 2 mm, and a thick-denier high-strength yarn was then wound thereon with a tension of 12 kg/N to corrugate the fabric. After the corrugating process, the core shaft with the composite fabric wound thereon was thermal set by placing it in hot water at a temperature of 90 °C for 5 min. After the corrugated shape of the fabric was stabilized, it was dried. Finally, artificial blood vessel with three-dimensional tubular form was obtained.

The artificial blood vessel prepared in Embodiment 1 has desirable softness. In a heart loop test, a loop height was measured as 10 cm. It is generally believed that a greater loop height indicates better softness. At a pressure of 16 kPa, its permeability was measured to be 150 ml/cm²•min. Generally, a permeability below 400 ml/cm²•min is considered to be low. Chemical analysis was performed on the artificial blood vessel prepared in Embodiment 1, according to Chinese national standard GB/T14233.1-2008, i.e., Test methods for Infusion, Transfusion, and Injection Equipment for Medical Use, Part 1: Chemical Analysis Methods. Further, biological test was also performed to the artificial blood vessel of Embodiment 1 in accordance with Chinese national standards GB/T 16886.4-2003, i.e., Biological Evaluation of Medical Devices, Part 4: Selection of Tests for Interactions with Blood, and GB/T 168865-2017, i.e., Biological Evaluation of Medical Devices, Part 5: Tests for in Vitro Cytotoxicity. The results of the chemical analysis and biological tests are summarized in Table 1 below.

**Table 1 Results of Chemical Analysis and Biological Performance Tests**

| | Test | Requirement | Result |
|---|---|---|---|
| Chemical analysis | Reducing substances | ≤ 2 ml | 0.15 ml |
| | Evaporation residue | ≤ 2 mg | 0.3 g |
| | Alkalinity/Acidity | ≤ 1.5 | 0.27 |
| | Content of heavy metals | A light color than the standard solution | Satisfied |
| | UV absorbance | <0.1 Abs | <0.1 Abs |
| Biological tests | Cell viability | ≥ 70% | 89% |
| | Hemolysis rate | < 5% | 0.0% |

As can be seen from Table 1, the artificial blood vessel prepared in Embodiment 1 meets all the chemical and biological performance requirements.

### Embodiment 2

The warp yarns were 11.11tex/72f (100D/72f) medical grade polytetrafluoroethylene yarns, which were multifilament yarns each composed of 72 monofilament yarns and had been sized. The weft yarns were multiple wound yarns with 6 monofilaments that were not twisted, each made of 4.44 tex (40D) polytetrafluoroethylene filaments. A composite fabrics were made of plain weaves and satin weaves with 5 long floating lines, wherein the area ratio of the plain weaves to the satin weaves was 1: 1. The composite fabric was further processed into a fabric tube with a diameter of 36 mm.

The appearance of the composite fabric is shown in Fig. 3, wherein black blocks represent warp interlacing points of the plain weave, gray blocks represent warp interlacing points of the satin weave. Moreover, the number of long warp floating lines was 5, and white blocks represent weft interlacing points. The Arabic numerals 1, 2, 3 ... 6 denote the plain weave, and the capital letters A, B, C ... F denote the satin weave.

The fabric for the artificial blood vessel was woven on a high-precision weaving machine and had a warp density of 480 yarns per 10 cm and a weft density of 300 yarns per 10 cm. The total number of the warp yarns was 544, each has 24 fibers, and warp beams of two systems operated simultaneously to feed the warp yarns. A sequential threading process followed, which involved threading the warp yarns through a total of 86 reed dents successively. During the weaving process, the yarns were weaved with even tension, and no breakage or pilling occurred. In this war, the resulting composite fabric had noticeable floats, a uniform and neat texture, and glossy and clean appearance.

The composite fabric was subject to desizing, corrugating and thermal setting processes in sequence. The desizing process was carried out at a temperature of 80 °C for 3 h. After the desizing proces, the composite fabric was disposed on a stainless steel core shaft with a pitch of 1 mm, and a thick-denier high-strength yarn was then wound thereon with a tension of 10 kg/N to corrugate the fabric. After the corrugating process, the core shaft with the composite fabric wound thereon was thermal-set by placing it in hot water at a temperature of 80 °C for 3 min. After the corrugated shape of the fabric was stabilized, it was dried. Finally, artificial blood vessel with three-dimensional tubular form was obtained.

The artificial blood vessel prepared in Embodiment 2 has desirable softness. In a heart loop test, a loop height was measured as 8 cm. It is generally believed that a greater loop height indicates better softness. At a pressure of 16 kPa, its permeability was measured to be 50 ml/cm²•min. It was further subjected to the same chemical analysis and biological performance tests as in Embodiment 1, and the test results met all the requirements.

### Embodiment 3

The warp yarns were 4.44tex/16f (40D/16f) medical grade polyethylene terephthalate yarns, which were multifilament yarns each composed of 16 monofilament yarns and had been sized. The weft yarns were twisted at a level of 100 turns per 10 cm. A composite fabric was made of plain weaves and satin weaves with 6 long floating lines, wherein the area ratio of the plain weaves to the satin weaves was 1: 1. The composite fabric was further processed into a fabric tube with a diameter of 8 mm.

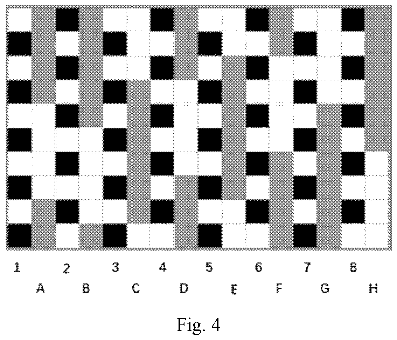

The appearance of the composite fabric is shown in Fig. 4, wherein black blocks represent warp interlacing points of the plain weave, gray blocks represent warp interlacing points of the satin weave. Moreover, the number of long warp floating lines was 6, and white blocks represent weft interlacing points. The Arabic numerals 1, 2, 3 ... 8 denote the plain weave, and the capital letters A, B, C ... H denote the satin weave.

The fabric for the artificial blood vessel was woven on a high-precision weaving machine, and had a warp density of 748 yarns per 10 cm and a weft density of 400 yarns per 10 cm. The total number of the warp yarns was 138, and warp beams of two systems operated simultaneously to feed the warp yarns. A sequential threading process followed, which involved threading the warp yarns through a total of 22 reed dents successively. During the weaving process, the yarns were weaved with even tension, and no breakage or pilling occurred. The resulting composite fabric had noticeable floating lines, a uniform and neat texture, and glossy and clean appearance.

The composite fabric was subject to desizing, corrugating and thermal setting processes in sequence. The desizing process was carried out at a temperature of 85 °C for 4 h. After the desizing process, the composite fabric was disposed on a stainless steel core shaft with a pitch of 1.5 mm, and a thick-denier high-strength yarn was then wound thereon with a tension of 11 kg/N to corrugate the fabric. After the corrugating process, the core shaft with the composite fabric wound thereon was thermal-set by placing it in hot water at a temperature of 85 °C for 4 min. After the corrugated shape of the fabric was stabilized, it was dried. Finally, artificial blood vessel with three-dimensional tubular form was obtained.

The artificial blood vessel prepared in Embodiment 3 has desirable softness. In a heart loop test, a loop height was measured as 16 cm. It is generally believed that a greater loop height indicates better softness. At a pressure of 16 kPa, its permeability was measured to be 200 ml/cm²•min. It was further subjected to the same chemical analysis and biological performance tests as in Embodiment 1, and the test results met all the requirements.

It is to be noted that although several artificial blood vessels and methods for preparing same have been described above as examples, it would be appreciated that, according to the present embodiment, the parameters such as yarn diameters/numbers and twist level may also be chosen from the scopes as mentioned above in the description. Therefore, the foregoing examples may be modified to obtain various alternatives, and the present invention is not limited thereto.

In conclusion, the artificial blood vessel and the method provided in the present application solve the problems of inadequate softness and excessive permeability of the conventional artificial blood vessels.

The description presented above is merely that of a few preferred embodiments of the present invention and is not intended to limit the scope thereof in any sense. Changes and modifications within the scope defined in the appended claims are possible.

## Claims

1. A method for preparing an artificial blood vessel, comprising:
weaving warp yarns and weft yarns to form interlaced first weaves and second weaves, both weaves extend along a weft direction to obtain a composite fabric; and
successively corrugating and thermal-setting the composite fabric to obtain the artificial blood vessel, wherein
the first weaves are plain weaves and the second weaves are satin weaves, each plain weave and each satin weave alternate with each other, and each of the satin weaves has warps floating over 5 to 9 wefts;
the composite fabric has a warp density in the range of 480 yarns per 10 cm to 748 yarns per 10 cm and a weft density of 280 yarns per 10 cm to 400 yarns per 10 cm.

2. The method of claim 1, wherein an area ratio of the first weave to the second weave is from 1: 1 to 1: 3.

3. The method of claim 1, wherein the warp and weft yarns are spun yarns made of polymer materials with biocompatibility.

4. The method of claim 3, wherein a material of the spun yarns is at least one selected from the group of polyethylene terephthalate, polyethylene, polytetrafluoroethylene and natural mulberry silk.

5. The method of claim 1, wherein the weft yarns are selected from the group of monofilament, multiple wound yarns and twisted yarns.

6. The method of claim 1, wherein there are two plain weaves located on either side of one satin weave, and there are two satin weaves located on either side of one plain weave.

7. The method of claim 1, wherein for each plain weave, the warp yarns and the weft yarns are interwoven with each other such that for two plain weaves on either side of each satin weave, the interweaving of the warp and weft yarns is opposite, and interweaving points of each yarn are exactly on opposite sides of respective interweaving points of each adjacent yarn.

8. An artificial blood vessel prepared by the method described in any of claims 1 to 7, comprising a composite fabric which is formed by first weaves and second weaves alternating along a weft direction, and the first and second weaves are weaved by warp yarns and weft yarns, wherein
the first weaves are plain weaves and the second weaves are satin weaves, each plain weave and each satin weave alternate with each other, and each of the satin weaves has warps floating over 5 to 9 wefts; the composite fabric has a warp density in the range of 480 yarns per 10 cm to 748 yarns per 10 cm and a weft density of 280 yarns per 10 cm to 400 yarns per 10 cm.

9. The artificial blood vessel of claim 8, wherein an area ratio of the first weave to the second weave is from 1: 1 to 1: 3.

10. The artificial blood vessel of claim 8, wherein there are two plain weaves located on either side of one satin weave, and there are two satin weaves located on either side of one plain weave.

11. The artificial blood vessel of claim 8, wherein for each plain weave, the warp yarns and the weft yarns are interwoven with each other such that for two plain weaves on either side of each satin weave, the interweaving of the warp and weft yarns is opposite, and interweaving points of each yarn are exactly on opposite sides of respective interweaving points of each adjacent yarn.

## Patentansprüche

1. Verfahren zur Herstellung eines künstlichen Blutgefäßes, umfassend:
Weben von Kettfäden und Schussfäden, um miteinander verflochtene erste und zweite Bindungen zu bilden, wobei beide Bindungen entlang einer Schussrichtung verlaufen, um ein Verbundgewebe zu erhalten; und
sukzessives Wellen und thermisches Fixieren des Verbundgewebes, um das künstliche Blutgefäß zu erhalten, wobei
die ersten Bindungen Leinwandbindungen sind und die zweiten Bindungen Satinbindungen sind, sich jede Leinwandbindung und jede Satinbindung jeweils abwechseln und jede der Satinbindungen Kettfäden hat, die über 5 bis 9 Schussfäden schweben; das Verbundgewebe eine Kettfadendichte im Bereich von 480 bis 748 Fäden pro 10 cm und eine Schussfadendichte von 280 bis 400 Fäden pro 10 cm aufweist.

2. Verfahren nach Anspruch 1, wobei ein Flächenverhältnis der ersten Bindung zu der zweiten Bindung 1:1 bis 1:3 beträgt.

3. Verfahren nach Anspruch 1, wobei die Kett- und Schussfäden gesponnene Fäden aus Polymermaterialien mit Biokompatibilität sind.

4. Verfahren nach Anspruch 3, wobei das Material der gesponnenen Fäden mindestens eines ist, das aus der Gruppe von Polyethylenterephthalat, Polyethylen, Polytetrafluorethylen und natürlicher Maulbeerseide ausgewählt ist.

5. Verfahren nach Anspruch 1, wobei die Schussfäden aus der Gruppe der Monofilamente, mehrfach gewickelten Fäden und gezwirnten Fäden ausgewählt werden.

6. Verfahren nach Anspruch 1, wobei sich auf beiden Seiten einer Satinbindung zwei Leinwandbindungen befinden und sich auf beiden Seiten einer Leinwandbindung zwei Satinbindungen befinden.

7. Verfahren nach Anspruch 1, wobei bei jeder Leinwandbindung die Kettfäden und die Schussfäden so miteinander verwebt werden, dass bei zwei Leinwandbindungen auf beiden Seiten jeder Satinbindung die Verwebung der Kett-und Schussfäden entgegengesetzt ist und die Verwebungspunkte jedes Fadens genau auf gegenüberliegenden Seiten der jeweiligen Verwebungspunkte jedes benachbarten Fadens liegen.

8. Ein künstliches Blutgefäß, das durch das in einem der Ansprüche 1 bis 7 beschriebene Verfahren hergestellt wird, umfassend ein Verbundgewebe, das aus ersten und zweiten Bindungen besteht, die sich entlang einer Schussrichtung abwechseln, und die ersten und zweiten Bindungen durch Kettfäden und Schussfäden gewebt sind, wobei
die ersten Bindungen Leinwandbindungen sind und die zweiten Bindungen Satinbindungen sind, sich jede Leinwandbindung und jede Satinbindung jeweils abwechseln und jede der Satinbindungen Kettfäden hat, die über 5 bis 9 Schussfäden schweben; das Verbundgewebe eine Kettfadendichte im Bereich von 480 bis 748 Fäden pro 10 cm und eine Schussfadendichte von 280 bis 400 Fäden pro 10 cm aufweist.

9. Das künstliche Blutgefäß nach Anspruch 8, wobei ein Flächenverhältnis der ersten Bindung zu der zweiten Bindung 1:1 bis 1:3 beträgt.

10. Das künstliche Blutgefäß nach Anspruch 8, wobei sich auf beiden Seiten einer Satinbindung zwei Leinwandbindungen befinden und sich auf beiden Seiten einer Leinwandbindung zwei Satinbindungen befinden.

11. Das künstliche Blutgefäß nach Anspruch 8, wobei bei jeder Leinwandbindung die Kettfäden und die Schussfäden so miteinander verwebt sind, dass bei zwei Leinwandbindungen auf beiden Seiten jeder Satinbindung die Verwebung der Kett-und Schussfäden entgegengesetzt ist und die Verwebungspunkte jedes Fadens genau auf gegenüberliegenden Seiten der jeweiligen Verwebungspunkte jedes benachbarten Fadens liegen.

## Revendications

1. Procédé de préparation d'un vaisseau sanguin artificiel, comprenant :
tisser des fils de chaîne et des fils de trame pour former des premiers et seconds tissages entrelacés, les deux tissages s'étendant le long d'une direction de trame pour obtenir un tissu composite ; et
ondulation et thermodurcissement successifs du tissu composite pour obtenir le vaisseau sanguin artificiel, dans lequel
les premières armures sont des armures simples et les secondes armures sont des armures satin, chaque armure simple et chaque armure satin alternent entre elles, et chacune des armures satin a des chaînes flottant sur 5 à 9 trames ; le tissu composite a une densité de chaîne dans la gamme de 480 fils par 10 cm à 748 fils par 10 cm et une densité de trame de 280 fils par 10 cm à 400 fils par 10 cm.

2. Procédé selon la revendication 1, dans lequel le rapport de surface du premier tissage au second tissage est compris entre 1: 1 et 1: 3.

3. Procédé selon la revendication 1, dans lequel les fils de chaîne et de trame sont des fils filés constitués de matériaux polymères présentant une biocompatibilité.

4. Procédé selon la revendication 3, dans lequel un matériau des fils filés est au moins un matériau choisi dans le groupe du polyéthylène téréphtalate, du polyéthylène, du polytétrafluoroéthylène et de la soie de mûrier naturelle.

5. Procédé selon la revendication 1, dans lequel les fils de trame sont choisis dans le groupe des fils monofilaments, des fils enroulés multiples et des fils torsadés.

6. Procédé selon la revendication 1, dans lequel il y a deux armures unies situées de chaque côté d'une armure satin, et il y a deux armures satin situées de chaque côté d'une armure unie.

7. Procédé selon la revendication 1, dans lequel, pour chaque armure toile, les fils de chaîne et les fils de trame sont entrelacés les uns avec les autres de telle sorte que pour deux armures toiles de chaque côté de chaque armure satin, l'entrelacement des fils de chaîne et de trame est opposé, et les points d'entrelacement de chaque fil sont exactement sur les côtés opposés des points d'entrelacement respectifs de chaque fil adjacent.

8. Vaisseau sanguin artificiel préparé selon le procédé décrit dans l'une quelconque des revendications 1 à 7, comprenant un tissu composite formé de premiers et seconds tissages alternés dans le sens de la trame, les premier et second tissages étant constitués de fils de chaîne et de fils de trame,
les premières armures sont des armures simples et les secondes armures sont des armures satin, chaque armure simple et chaque armure satin alternent entre elles, et chacune des armures satin a des chaînes flottant sur 5 à 9 trames ; le tissu composite a une densité de chaîne dans la gamme de 480 fils par 10 cm à 748 fils par 10 cm et une densité de trame de 280 fils par 10 cm à 400 fils par 10 cm.

9. Vaisseau sanguin artificiel selon la revendication 8, dans lequel le rapport de surface du premier tissage au second tissage est de 1: 1 à 1: 3.

10. Vaisseau sanguin artificiel selon la revendication 8, dans lequel il y a deux armures unies situées de chaque côté d'une armure satin, et il y a deux armures satin situées de chaque côté d'une armure unie.

11. Vaisseau sanguin artificiel selon la revendication 8, dans lequel, pour chaque armure toile, les fils de chaîne et les fils de trame sont entrelacés les uns avec les autres de telle sorte que pour deux armures toiles de chaque côté de chaque armure satin, l'entrelacement des fils de chaîne et de trame est opposé, et les points d'entrelacement de chaque fil sont exactement sur les côtés opposés des points d'entrelacement respectifs de chaque fil adjacent.
